# EUROPÄISCHE PATENTSCHRIFT

(11) **EP 3 125 741 B1**
(45) Veröffentlichungstag und Bekanntmachung des Hinweises auf die Patenterteilung: **10.06.2020**
(21) Anmeldenummer: 15712834.9
(22) Anmeldetag: 18.03.2015
(51) Int. Cl.: A61L 2/18, A61B 17/00, A61B 90/70, A61B 1/00, A61B 1/12

(54) **VERBINDUNGSEINRICHTUNG, REINIGUNGS- UND/ODER DESINFIZIEREINRICHTUNG UND VERFAHREN ZUM BETREIBEN DERSELBEN**
CONNECTING DEVICE, CLEANING AND/OR DISINFECTING DEVICE, AND METHOD FOR THE OPERATION THEREOF
DISPOSITIF DE RACCORDEMENT, DISPOSITIF DE NETTOYAGE ET/OU DE DÉSINFECTION ET PROCÉDÉ SERVANT À FAIRE FONCTIONNER LESDITS DISPOSITIFS

(30) Priorität: 31.03.2014 DE 102014206020
(43) Veröffentlichungstag der Anmeldung: 08.02.2017
(73) Patentinhaber: Olympus Winter & Ibe GmbH, 22045 Hamburg (DE)
(72) Erfinder: OTTENS, Benjamin, 22309 Hamburg (DE)
(74) Vertreter: Seemann & Partner Patentanwälte mbB
(86) Internationale Anmeldenummer: PCT/EP2015/055692
(87) Internationale Veröffentlichungsnummer: WO 2015/150080

(56) Entgegenhaltungen:
- EP-A1- 2 098 185
- DE-A1- 3 737 121
- JP-A- 2004 135 946
- US-A- 4 042 248
- US-A- 5 083 802
- US-A- 5 931 647

## Beschreibung

Die Erfindung betrifft eine Verbindungseinrichtung zum Ankoppeln eines Spülsystems an einen zu reinigenden Kanal eines chirurgischen Instruments, insbesondere einen Endoskopkanal eines Endoskops, wobei die Verbindungseinrichtung eine versorgungsseitige Kopplungsvorrichtung mit einem Aufnahmekanal umfasst, der dazu eingerichtet ist, ein Verbindungselement aufzunehmen, welches sich im verbundenen Zustand in einer Längsaxialrichtung des Aufnahmekanals innerhalb des Aufnahmekanals erstreckt und welches endseitig eines Verbindungskanals vorgesehen ist, der mit dem zu reinigenden Kanal des chirurgischen Instruments kommuniziert, wobei der Aufnahmekanal zur Zuführung von Spülflüssigkeit zu dem zu reinigenden Kanal mit dem Spülsystem verbunden ist, wobei die Kopplungsvorrichtung ein Dichtelement umfasst.

Außerdem betrifft die Erfindung eine Reinigungs- und/oder Desinfiziereinrichtung zur Reinigung und/oder Desinfektion von chirurgischen Instrumenten, insbesondere Endoskopen, mit einem zu reinigenden Kanal, insbesondere zur Reinigung und/oder Desinfektion von Endoskopen mit einem zu reinigenden Endoskopkanal, umfassend einen Reinigungsraum, in dem die zu reinigenden und/oder zu desinfizierenden chirurgischen Instrumente, insbesondere Endoskope, während eines Reinigungs- und/oder Desinfektionsvorgangs aufnehmbar sind oder aufgenommen werden.

Schließlich betrifft die Erfindung ein Verfahren zum Betreiben einer solchen Reinigungs- und/oder Desinfiziereinrichtung.

An die Aufbereitung von Endoskopen nach Benutzung in einem Reinigungs- und Desinfektionsgerät für die Endoskopie, abgekürzt als RDG-E, werden im klinischen Bereich hohe Anforderungen gestellt. Die Aufbereitung umfasst typischerweise die Schritte: Waschen, Desinfizieren und Trocknen der Endoskope. Der Desinfizierung gehen ein oder zwei Wasch- oder Vorwaschgänge voraus. Anschließend erfolgen Spülgänge mit klarem Wasser und Trocknungsgänge. Zum Waschen und Desinfizieren werden dem Wasch- bzw. Reinigungsmittel eine oder mehrere Chemikalien zum Desinfizieren hinzudosiert.

Besondere Bedeutung bei der Aufbereitung von Endoskopen kommt der Reinigung und Desinfektion der Endoskopkanäle zu. Zur Aufbereitung des Endoskops werden diese Kanäle mit dem Spülkreislauf der RDG-E verbunden. Die Verbindung erfolgt über spezielle Verbindungselemente. Die Verbindung zwischen Endoskop und Verbindungselement wird vielfach manuell hergestellt. Zwischen dem Verbindungselement und der Aufbereitungsvorrichtung ist es jedoch wünschenswert, dass die Verbindung automatisch hergestellt wird. Je nach Typ und Aufbau der RDG-E ist eine manuelle Verbindung mit dem oder den Verbindungselement(n) unhandlich durchzuführen, zeitraubend und außerdem fehleranfällig.

Eine Aufbereitungsvorrichtung ist beispielsweise unter der Bezeichnung ETD 3 des Herstellers Olympus Medical Systems bekannt, wobei die Abkürzung "ETD" für "Endo Thermo Disinfector" steht. Diese Aufbereitungsvorrichtung ist mit verschiedenen Aufbereitungsprogrammen ausgestattet und erlaubt die simultane Aufbereitung mehrerer flexibler oder starrer Endoskope. In den ETD Maschinen erfolgt eine automatische Ankopplung zwischen den Verbindungselementen und dem Spülkreislauf der Maschine.

In der ETD 3 werden die Verbindungselemente, die mit den zu reinigenden Endoskopen verbunden sind, durch Einschieben des Reinigungskorbs, der die zu reinigenden Endoskope aufnimmt, an der Rückwand des Spülraums passiv angekoppelt. Alternativ erfolgt die Ankopplung über ein seitlich in dem Spülraum vorhandenes Modul, welches eine Verbindungselementplatte mit Hilfe eines Motors aktiv mit der ETD verbindet.

Bei passiven Ankoppelverfahren muss ein mechanischer Widerstand überwunden werden, da beim Einschieben der Verbindungskupplung eine geräteseitige Dichtsituation hergestellt werden muss. Außerdem ist vielfach eine Verriegelung notwendig, was zusätzlichen Kraftaufwand bzw. weitere Arbeitsschritte durch den Anwender erfordert. Die mechanische Überwindung der Dichtsituation führt außerdem zu einem erhöhten Verschleiß. Hinzu kommt, dass die seitens des Anwenders aufzubringende Kraft vielfach zumindest teilweise auch auf die Wand des Spülraums einwirkt. Dies zieht, vor allem bei geringen Wandstärken, unerwünschte dauerhafte Verformungen nach sich.

Aktive Ankoppelverfahren (z.B. unter Verwendung von Motoren) sind deutlich teurer und beanspruchen einen nicht unerheblichen Bauraum in der Aufbereitungsvorrichtung. Hinzu kommt, dass aktiv angetriebene Systeme vielfach wartungsintensiv sind.

Aus der EP 2 098 185 A1 ist ein Anschlussstück bekannt, wie es am endoskopseitigen Ende eines Verbindungsschlauchs einer Aufbereitungs- und Desinfektionseinrichtung vorgesehen ist. Dieses Anschlussstück wird auf ein endoskopseitiges Endstück aufgesetzt.

Ferner ist aus der US 5,931,647 A eine Dosierpumpe bekannt, welche möglichst einfach aufgebaut und kostengünstig herstellbar sein soll.

Aus JP 2004 135 946 ist eine Vorrichtung zur Reinigung und Desinfektion von Endoskopen bekannt. Ein Endoskop wird in einen Reinigungsbehälter eingelegt und über Verbindungsstücke, Flüssigkeitsschläuche und Luftzufuhrschläuche mit Reinigungsflüssigkeit und Druckluft beaufschlagt. Die Zufuhr von Reinigungsmittel geschieht über ein Anschlussstück. Um eine Verbindung abzudichten, ist ein aufblasbares Versiegelungselement vorgesehen, welches mit Druckluft beaufschlagt werden kann, um einen dichten Sitz zu gewährleisten.

DE 37 37 121 zeigt ein Abdichtsystem, welches bei einem operativen Eingriff in ein Hohlorgan verwendet wird. Es ist eine pneumatisch aufblasbare Manschette vorgesehen, durch die ein Schlauch geführt werden kann, und welche sich an den Schlauch anlegt, um einen Dichtsitz zu gewährleisten.

Ausgehend von diesem Stand der Technik ist es eine Aufgabe der Erfindung, eine Verbindungseinrichtung, eine Reinigungs- und/oder Desinfiziereinrichtung und ein Verfahren zum Betreiben einer Reinigungs- und/oder Desinfiziereinrichtung anzugeben, wobei eine zuverlässige und möglichst verschleißarme Ankopplung eines zu reinigenden Kanals mit einem Spülsystem bereitstellbar sein soll und wobei insbesondere der konstruktive Aufwand möglichst gering gehalten werden soll.

Die Aufgabe wird gelöst durch eine Verbindungseinrichtung zum Ankoppeln eines Spülsystems an einen zu reinigenden Kanal eines chirurgischen Instruments, insbesondere einen Endoskopkanal eines Endoskops, wobei die Verbindungseinrichtung eine versorgungsseitige Kopplungsvorrichtung mit einem Aufnahmekanal umfasst, der dazu eingerichtet ist, ein Verbindungselement aufzunehmen, welches sich im verbundenen Zustand in einer Längsaxialrichtung des Aufnahmekanals innerhalb des Aufnahmekanals erstreckt und welches endseitig eines Verbindungskanals vorgesehen ist, der mit dem zu reinigenden Kanal des chirurgischen Instruments kommuniziert, wobei der Aufnahmekanal zur Zuführung von Spülflüssigkeit zu dem zu reinigenden Kanal mit dem Spülsystem verbunden ist, wobei die Kopplungsvorrichtung ein Dichtelement umfasst, wobei die Verbindungseinrichtung dadurch fortgebildet ist, dass das Dichtelement elastisch ist und Mittel zur hydraulischen und/oder pneumatischen Betätigung des Dichtelements vorgesehen sind, wobei das hydraulisch und/oder pneumatisch betätigte Dichtelement durch eine Expansion quer zu der Längsaxialrichtung einen Querschnitt des Aufnahmekanals im Bereich des Dichtelements verringert, so dass eine flüssigkeitsdichte Verbindung des Verbindungselements mit der Kopplungsvorrichtung bereitstellbar ist oder bereitgestellt wird, wobei
eine Überdruckkammer und eine Druckluftversorgung vorgesehen sind, wobei die Druckluftversorgung mit der Überdruckkammer verbunden ist und das Dichtelement zumindest einen Wandabschnitt der Überdruckkammer bildet,

die Kopplungsvorrichtung die Überdruckkammer umfasst, wobei sich die Überdruckkammer entlang einem Außenumfang des Aufnahmekanals erstreckt und wobei das Dichtelement eine Dichtmembran ist, die die Überdruckkammer an einer dem Aufnahmekanal zugewandten Innenwand der Überdruckkammer begrenzt und
ein äußeres Bauteil der Kopplungsvorrichtung eine mit einer Druckluftversorgung verbundene Überdruckkammer umfasst, wobei die Innenwand der Überdruckkammer entlang dem Umfang des Aufnahmekanals ringförmig unterbrochen ist, so dass die Innenwand einen ringförmigen Spalt aufweist, wobei der ringförmige Spalt mit der elastischen Dichtmembran als Dichtelement verschlossen ist.

Der Erfindung liegt die Erkenntnis zugrunde, dass eine flüssigkeitsdichte Verbindung zwischen einer Kopplungsvorrichtung und einem Verbindungselement unter Verwendung eines hydraulisch und/oder pneumatisch betätigten Dichtelements verschleißarm arbeitet, praktisch keinen zusätzlichen Kraftaufwand zum Ankoppeln des Verbindungselements an die Kopplungsvorrichtung erfordert und außerdem konstruktiv einfach zu realisieren ist. Die hydraulisch und/oder pneumatisch betätigbare Dichtung gewährleistet eine zuverlässig flüssigkeitsdichte, hygienisch einwandfreie Dichtsituation zwischen einem Verbindungskanal des Verbindungselements und dem zu reinigenden Kanal des chirurgischen Instruments. Die Reinigungs- und/oder Desinfektionsflüssigkeit tritt mit einem Außenraum nicht in Kontakt, so dass der zu reinigende Kanal zuverlässig und hygienisch spülbar ist.

Erfindungsgemäß ist die Verbindungseinrichtung dadurch fortgebildet, dass eine Überdruckkammer und eine Druckluftversorgung vorgesehen sind, wobei die Druckluftversorgung mit der Überdrucckammer verbunden ist und das Dichtelement zumindest einen Wandabschnitt der Überdruckkammer bildet.

Das Dichtelement wird expandiert, indem die Überdruckkammer mit Druckluft beaufschlagt wird. Insbesondere wird die Überdruckkammer mit einem Überdruck beaufschlagt. Als Überdruck wird ein gegenüber dem Atmosphärendruck erhöhter Druck bezeichnet. Dieser Überdruck führt dazu, dass sich das Dichtelement elastisch verformt und soweit expandiert, dass eine zuverlässige Dichtsituation zwischen der Kopplungsvorrichtung und dem Verbindungselement vorliegt.

Das Dichtelement ist insbesondere als separates Bauteil vorgesehen. Beispielsweise wird eine schlauchförmige Dichtung, insbesondere aus einem gummielastischen Material, beispielsweise aus Gummi oder Silikon, verwendet, deren Innenraum die Überdrucckammer ist. Eine solche schlauchförmige Dichtung bzw. ein solcher Dichtungsschlauch wird durch Anlegen eines Überdrucks in seinem Innenraum expandiert, so dass sein Querschnitt zunimmt. Diese Ausführung der Verbindungseinrichtung ist besonders kostengünstig.

Erfindungsgemäß ist ferner vorgesehen, dass die Kopplungsvorrichtung die Überdruckkammer umfasst, wobei sich die Überdruckkammer entlang eines Außenumfangs des Aufnahmekanals erstreckt und wobei das Dichtelement eine Dichtmembran ist, die die Überdruckkammer an einer dem Aufnahmekanal zugewandten Innenwand der Überdruckkammer begrenzt.

Insbesondere ist die Überdruckkammer in die Kopplungsvorrichtung integriert, ist also mit anderen Worten ein integraler Bestandteil der Kopplungsvorrichtung.

Vorteilhaft ist die Kopplungsvorrichtung einschließlich der Überdruckkammer schnell und kostengünstig als ein gemeinsames Bauteil, beispielsweise in einem Spritzgussverfahren, herstellbar. Das Dichtelement ist durch seine Anordnung an der Innenwand der Überdruckkammer vor unbeabsichtigten oder versehentlichen Beschädigungen weitgehend geschützt. Dies erhöht die Zuverlässigkeit und Haltbarkeit der Verbindungseinrichtung.

Ferner ist die Verbindungseinrichtung dadurch fortgebildet, dass die Überdruckkammer vollständig entlang dem Umfang des Aufnahmekanals verläuft, wobei durch Beaufschlagen der Überdruckkammer mit einem Überdruck eine flüssigkeitsdichte Ankopplung des Verbindungselements an die Kopplungsvorrichtung bereitstellbar ist oder bereitgestellt wird, indem ein Dichtsitz zwischen dem expandierten, die Innenwand der Überdruckkammer überragenden und so den Querschnitt des Aufnahmekanals lokal reduzierenden Dichtelements und einer Außenseite eines sich innerhalb des Aufnahmekanals erstreckenden Anschlussstutzens des Verbindungselements herstellbar ist oder hergestellt wird.

Erfindungsgemäß ist vorgesehen, dass ein äußeres Bauteil der Kopplungsvorrichtung eine mit einer Druckluftversorgung verbundene Überdruckkammer umfasst, wobei die Innenwand der Überdrucckammer entlang dem Umfang des Aufnahmekanals ringförmig unterbrochen ist, so dass die Innenwand einen ringförmigen Spalt aufweist, wobei der ringförmige Spalt mit der elastischen Dichtmembran als Dichtelement verschlossen ist.

Wird das Dichtelement bei pneumatischer Betätigung expandiert, stellt es, ausgehend von der Innenwand der Überdruckkammer, einen Dichtsitz mit der Außenseite des Anschlussstutzens des Verbindungselements her.

Ein in eine Trennwand zwischen der Überdruckkammer und dem Aufnahmekanal eingelassenes Dichtelement, insbesondere eine elastische Dichtmembran, beispielsweise eine Membran aus einem gummielastischen Material wie beispielsweise Gummi oder Silikon, ist vor Beschädigungen, auch bei Einführen des Verbindungselements in die Kopplungsvorrichtung, weitgehend geschützt. Eine versehentliche Beschädigung des Dichtelements ist so praktisch ausgeschlossen, dies erhöht die Zuverlässigkeit und Haltbarkeit der Verbindungseinrichtung.

Die erfindungsgemäße Aufgabe wird ferner gelöst durch eine Reinigungs- und/oder Desinfiziereinrichtung zur Reinigung und/oder Desinfektion von chirurgischen Instrumenten, insbesondere Endoskopen, mit einem zu reinigenden Kanal, insbesondere zur Reinigung und/oder Desinfektion von Endoskopen mit einem zu reinigenden Endoskopkanal, umfassend einen Reinigungsraum, in dem die zu reinigenden und/oder zu desinfizierenden chirurgischen Instrumente, insbesondere Endoskope, während eines Reinigungs- und/oder Desinfektionsvorgangs aufnehmbar sind oder aufgenommen werden, wobei die Reinigungs- und/oder Desinfiziereinrichtung dadurch fortgebildet ist, dass eine Verbindungseinrichtung nach einer oder mehreren der genannten Ausführungsformen vorgesehen ist, die insbesondere an einer Wand des Reinigungsraums angeordnet oder in eine Wand des Reinigungsraums eingelassen ist.

Insbesondere umfasst die Reinigungs- und/oder Desinfiziereinrichtung ein Spülsystem, welches neben einem Spülflüssigkeitsvorrat, der beispielsweise ein Reinigungs- und/oder Desinfektionsmittel zur Reinigung und/oder Desinfektion des zu reinigenden Kanals, insbesondere Endoskopkanals, des chirurgischen Instruments, insbesondere Endoskops, umfasst, Pumpen, Sensoren und Ventile umfasst.

Gleiche oder ähnliche Vorteile, wie sie bereits im Hinblick auf die Verbindungseinrichtung erwähnt wurden, treffen auch auf die Reinigungs- und/oder Desinfiziereinrichtung zu und sollen nicht wiederholt werden.

Die erfindungsgemäße Aufgabe wird schließlich gelöst durch ein Verfahren zum Betreiben einer Reinigungs- und/oder Desinfiziereinrichtung nach einer oder mehreren der genannten Ausführungsformen, wobei das Verfahren die folgenden Verfahrensschritte umfasst:
- Einbringen eines Anschlussstutzens des Verbindungselements entlang der Längsaxialrichtung des Aufnahmekanals in den Aufnahmekanal der versorgungsseitigen Kopplungsvorrichtung,
- Anlegen eines Überdrucks an das Dichtelement durch Beaufschlagen des Dichtelements mit einem unter Überdruck stehenden hydraulisch und/oder pneumatisch wirksamen Fluid zur hydraulischen und/oder pneumatischen Betätigung des Dichtelements, so dass das betätigte Dichtelement durch eine Expansion quer zu der Längsaxialrichtung einen Querschnitt des Aufnahmekanals im Bereich des Dichtelements verringert,
- Beaufschlagen des Aufnahmekanals mit einer Spülflüssigkeit, so dass die Spülflüssigkeit ausgehend von dem Aufnahmekanal über den Verbindungskanal zum Reinigen und/oder zum Desinfizieren des zu reinigenden Kanals des chirurgischen Instruments, insbesondere zum Reinigen und/oder zum Desinfizieren eines Endoskopkanals eines Endoskops, zur Verfügung steht,
- Entlasten des hydraulisch und/oder pneumatisch wirksamen Fluids, um den durch das Fluid auf das Dichtelement wirkenden Überdruck abzubauen, so dass das Verbindungselement von der Kopplungsvorrichtung freigegeben wird.

Insbesondere wird der Verbindungselement manuell mit dem zu reinigenden chirurgischen Instrument, insbesondere dem Endoskop, verbunden. Ferner sind der oder die Anschlussstutzen des oder der Verbindungselement(e), die anschließend mit der Kopplungsvorrichtung verbunden werden, insbesondere lösbar oder dauerhaft mit einem Reinigungskorb zur Aufnahme des chirurgischen Instruments befestigt. Ein solcher Reinigungskorb wird in einen Reinigungsraum der Reinigungs- und/oder Desinfiziereinrichtung eingeschoben, wobei vorzugsweise im Zuge dieser Bewegung der Anschlussstutzen des Verbindungselements entlang der Längsaxialrichtung des Aufnahmekanals in den Aufnahmekanal der versorgungsseitigen Kopplungsvorrichtung eingebracht wird.

Vorteilhaft erfolgt diese Bewegung, ohne dass hierzu der Widerstand einer Dichtsituation überwunden werden muss. Ebenso entfällt ein zusätzlicher Handgriff zum Verriegeln des Verbindungselements.

Insbesondere befindet sich die Kopplungsvorrichtung an einer Rückwand des Behandlungsraums. Sie ist passend zu den Verbindungselementen positioniert und insbesondere so mit den Führungen bzw. Anschlägen des Reinigungskorbs abgestimmt, dass beim Einschieben des Reinigungskorbs ein Anschlussstutzen des Verbindungselements in den Aufnahmekanal der Kopplungsvorrichtung eingeschoben wird.

Ferner ist insbesondere ein definierter Anschlag vorgesehen, um dem Anwender zu signalisieren, dass sich der Reinigungskorb in der Endposition befindet.

In der Endposition ist noch kein flüssigkeitsdichter Kontakt zwischen der Kopplungsvorrichtung und dem Verbindungselement hergestellt. Insbesondere ist kein besonderer Kraftaufwand notwendig, um die Endposition zu erreichen und die Verbindung zwischen Verbindungselement und Kopplungsvorrichtung herzustellen.

Schließt der Anwender anschließend die Reinigungs- und/oder Desinfiziereinrichtung und wählt ein entsprechendes Reinigungs- und/oder Desinfektionsprogramm, so wird das Dichtelement hydraulisch und/oder pneumatisch betätigt, wobei dieses insbesondere elastisch expandiert. Bevorzugt wird einer Überdruckkammer Druckluft zugeführt.

Das Dichtelement bzw. die Dichtmembran verformt sich soweit, dass zwischen einer Außenseite des Verbindungselements, insbesondere einer Außenseite des Anschlussstutzens, und dem Dichtelement eine zuverlässige Dichtsituation hergestellt ist. Der zu reinigende Kanal des chirurgischen Instruments, insbesondere der Endoskopkanal, ist nun mit Spülflüssigkeit beaufschlagbar. Er wird mit aus dem Spülflüssigkeitsvorrat entnommener Spülflüssigkeit gespült. Bei der Spülflüssigkeit handelt es sich beispielsweise um Wasser und/oder um eine Reinigungs- und/oder Desinfektionslösung. Insbesondere ist das Wasser und/oder die Reinigungs- und/oder Desinfektionslösung auf oder über eine vorgebbare Temperatur erhitzt.

Gemäß einer weiteren Ausführungsform ist das erfindungsgemäße Verfahren dadurch fortgebildet, dass die folgenden weiteren Schritte vorgesehen sind
- Zuführen von Druckluft zu einer zumindest teilweise von dem Dichtelement umschlossenen Überdruckkammer, wobei ein Zuführdruckniveau der Druckluft durch Zuschalten oder Abschalten einer Druckluftversorgung verändert wird,
- Messen eines zeitabhängigen Drucks in der Überdruckkammer und Vergleichen des gemessenen Drucks mit einem vorgegebenen Solldruck, wobei durch Zuschalten oder Abschalten der Druckluftversorgung der Solldruck für eine Dauer des Reinigungs- und/oder Desinfiziervorgangs aufrechterhalten wird.

Ferner ist bevorzugt vorgesehen, dass der Druck in der Überdrucckammer an einem mit einer Druckluftversorgung verbundenen Zugang der Überdruckkammer über einen ersten Zeitraum gemessen wird, wobei anschließend das Zuführdruckniveau stufenförmig verändert wird und eine Sprungantwort des in der Überdruckkammer herrschenden Drucks auf das Verändern des Zuführdruckniveaus gemessen und analysiert wird, wobei die Analyse Auskunft über eine Dichtigkeit der Überdruckkammer, insbesondere eine Dichtigkeit des Dichtelements, und/oder Auskunft über ein Vorhandensein oder Nicht-Vorhandensein des Anschlussstutzens des Verbindungselements in dem Aufnahmekanal gibt.

Gemäß einer weiteren Ausführungsform ist vorgesehen, dass die Analyse das Bilden eines Integrals des gemessenen Drucks über eine vorgebbare Zeit, das Bestimmen einer Steigung und/oder das Bilden einer zweiten Ableitung des als Sprungantwort gemessenen Drucks nach der Zeit, beinhaltet, wobei ein Vergleich mit einem vorgegebenen Sollwert durchgeführt wird und bei Über- oder Unterschreiten des vorgegebenen Sollwerts auf eine nicht ausreichende Dichtigkeit der Überdruckkammer, insbesondere des Dichtelements, und/oder auf ein Vorhandensein oder Nicht-Vorhandensein des Anschlussstutzens des Verbindungselements in dem Aufnahmekanal geschlossen wird.

Mit anderen Worten wird der Druck während der Dauer des Reinigungsprogramms aufrechterhalten. Eine geringe Leckrate wird kompensiert.

Um ein beschädigtes Dichtelement und/oder ein Vorhandensein oder Nicht-Vorhandensein des Anschlussstutzens des Verbindungselements in dem Aufnahmekanal zu detektieren, wird das Zuführdruckniveau stufenförmig verändert und eine Dichtigkeit der Überdruckkammer bzw. ein elastisches Verhalten des Dichtelements anhand einer Sprungantwort auf das Verändern des Zuführdruckniveaus gemessen und analysiert.

Die Analyse umfasst insbesondere einen Vergleich eines statischen Drucks mit einem für die Überdruckkammer vorgebbaren ersten Wert.

Ferner umfasst die Analyse insbesondere das Bilden eines Integrals des gemessenen Drucks über eine zweite vorgebbare Zeit und einen Vergleich mit einem für die Überdruckkammer vorgebbaren zweiten Wert.

Außerdem umfasst die Analyse insbesondere das Bestimmen einer Steigung und/oder das Bilden einer zweiten Ableitung des gemessenen Drucks nach der Zeit.

Schließlich umfasst die Analyse insbesondere die Analyse einer Dauer, Amplitude und/oder Wellenlänge einer Schwingung des als Sprungantwort gemessenen Drucks.

Übersteigt der Druckabfall einen gewissen Grenzwert, so wird dies als Hinweis auf ein defektes Dichtelement gewertet, wobei insbesondere ein entsprechendes Fehler- oder Hinweissignal ausgegeben wird. Hierzu werden insbesondere in Bezug auf das gebildete Integral über einer zweiten vorgebbaren Zeit, die gebildete Steigung und/oder zweite Ableitung des gemessenen Drucks nach der Zeit sowie die Dauer, Amplitude und/oder Wellenlänge einer Schwingung des als Sprungantwort gemessenen Drucks Grenzwerte bestimmt, deren Über- bzw. Unterschreiten eine zu hohe Leckrate anzeigen.

Vorteilhaft ist so die Funktionsfähigkeit des Dichtelements zuverlässig überprüfbar.

Das Vorhandensein oder Nicht-Vorhandensein des Anschlussstutzens des Verbindungselements in dem Aufnahmekanal wird anhand der mechanischen bzw. elastischen Reaktion des Dichtelements auf den veränderten Innendruck in der Überdruckkammer detektiert. Das Dichtelement zeigt ein unterschiedliches elastisches Verhalten, abhängig davon, ob der Anschlussstutzen in dem Aufnahmekanal vorhanden ist oder nicht. Beispielsweise übt der Anschlussstutzen eine entsprechende Gegenkraft auf das Dichtelement aus und begrenzt außerdem das zur elastischen Verformung bzw. Ausdehnung zur Verfügung stehende Volumen. Insbesondere durch eine Analyse einer Steigung und/oder das Bilden einer zweiten Ableitung des gemessenen Drucks nach der Zeit kann festgestellt werden, ob sich der Anschlussstutzen in dem Aufnahmekanal befindet. Ferner bietet sich zu diesem Zweck eine Analyse einer Dauer, einer Amplitude und/oder einer Wellenlänge einer Schwingung des als Sprungantwort gemessenen Drucks an.

Nach Beendigung des Reinigungsprozesses wird der Druckluftkanal entlüftet, wodurch das Verbindungselement von der Kopplungsvorrichtung freigegeben wird. Erneut ist der Anwender in der Lage, den Korb mit den enthaltenen gereinigten chirurgischen Instrumenten zu entnehmen, ohne dass ein Kraftaufwand erforderlich ist, um eine Dichtsituation zu überwinden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1 bis 4: schematisch eine Verbindungseinrichtung in einer Längsschnittansicht während verschiedener Betriebszustände und
- Fig. 5: eine schematische Reinigungs- und Desinfiziereinrichtung.

In den Zeichnungen sind jeweils gleiche oder gleichartige Elemente und/oder Teile mit denselben Bezugsziffern versehen, so dass von einer erneuten Vorstellung jeweils abgesehen wird.

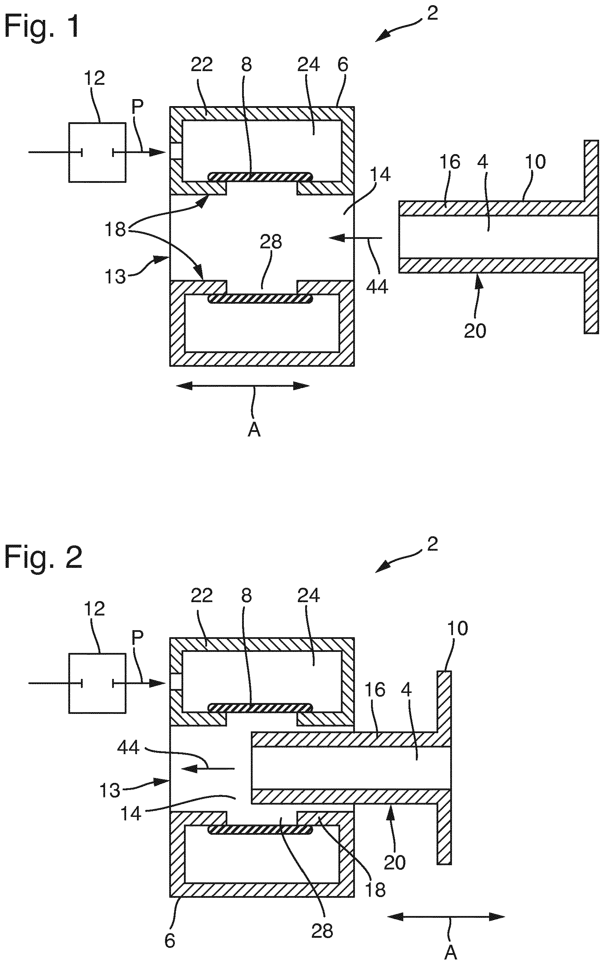

Die Fig. 1 bis 4 zeigen schematisch eine Verbindungseinrichtung 2 in einer Längsschnittansicht in verschiedenen Betriebszuständen. Die Verbindungseinrichtung 2 umfasst eine Kopplungsvorrichtung 6 und ein Verbindungselement 10, das mit der Kopplungsvorrichtung 6 verbindbar ist. Die Kopplungsvorrichtung 6 ist versorgungsseitig vorhanden und dient der Bereitstellung einer Spülflüssigkeit 5, beispielsweise Wasser oder einer Reinigungs- und/oder Desinfektionsflüssigkeit, zur Reinigung und/oder Desinfektion eines zu reinigenden Kanals eines chirurgischen Instruments, insbesondere eines Endoskopkanals eines Endoskops.

Zu diesem Zweck ist die Kopplungsvorrichtung 6, genauer der Aufnahmekanal 14 der Kopplungsvorrichtung 6, über eine Versorgungsöffnung 13 mit einem Spülsystem 50 verbunden. Der Verbindungskanal 4 des Verbindungselements 10 ist mit dem zu reinigenden Kanal des chirurgischen Instruments verbunden.

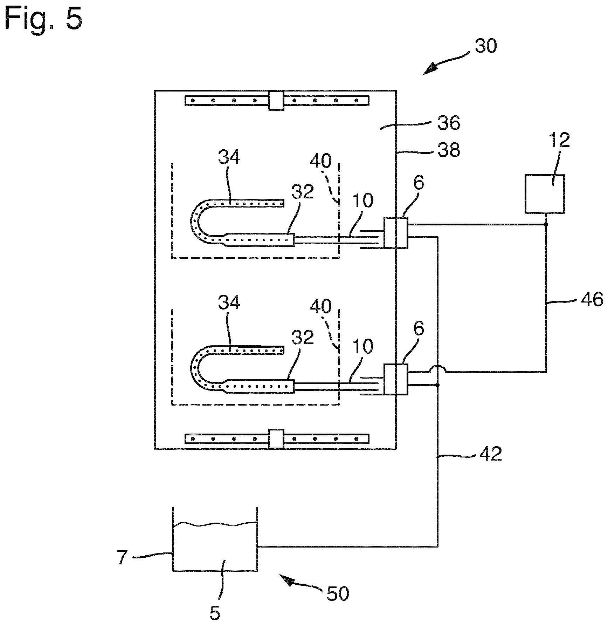

Fig. 5 zeigt eine Reinigungs- und/oder Desinfiziereinrichtung 30, in die die Kopplungsvorrichtung 6 integriert ist. Sie befindet sich beispielhaft an einer Rückwand 38 eines Reinigungsraums 36. Die Reinigungs- und/oder Desinfiziereinrichtung 30 dient der Reinigung und/oder Desinfektion von chirurgischen Instrumenten mit einem zu reinigenden Kanal. Beispielhaft ist eine Reinigungs- und/oder Desinfiziereinrichtung 30 zur Reinigung und/oder Desinfektion von Endoskopen 32 mit einem zu reinigenden Endoskopkanal 34 dargestellt.

Die Versorgungsöffnung 13 (vgl. Fig. 1 bis 4) ist mit dem Spülflüssigkeitsvorrat 7 über eine Versorgungsleitung 42 verbunden (vgl. Fig. 5), so dass die Spülflüssigkeit 5 der Kopplungsvorrichtung 6 über deren Versorgungsöffnung 13 zugeführt werden kann. Das Spülsystem 50 umfasst neben dem Spülflüssigkeitsvorrat 7 nicht dargestellte Pumpen, Sensoren und Ventile.

Das Verbindungselement 10 ist mit dem zu reinigenden Endoskop 32 verbunden. Der in dem Verbindungselement 10 vorhandene Verbindungskanal 4 kommuniziert also mit einem zu reinigenden Endoskopkanal 34. Nachdem die Verbindung zwischen der Kopplungsvorrichtung 6 und dem Verbindungselement 10 hergestellt ist, wird der Kanal mit Spülflüssigkeit 5, beispielsweise einer Reinigungs- und/oder Desinfektionsflüssigkeit, aus dem Spülflüssigkeitsvorrat 7 gespült.

Zu Beginn des Reinigungsvorgangs wird das Verbindungselement 10 bevorzugt manuell mit dem zu reinigenden Endoskop 32 verbunden. Der Anschlussstutzen 16 des verwendeten Verbindungselements ist insbesondere lösbar oder dauerhaft an einem Reinigungskorb 40 zur Aufnahme des Endoskops 32 befestigt.

Schiebt der Anwender den Reinigungskorb 40 einschließlich der zu reinigenden Endoskope 32 in einen Reinigungsraum 36 der Reinigungs- und/oder Desinfiziereinrichtung 30, so erfolgt im Zuge dieser Bewegung das Einführen der Anschlussstutzen 16 in den Aufnahmekanal 14 der Kopplungsvorrichtung 6 in einer Verbindungsrichtung 44. Die Verbindungsrichtung ist zumindest näherungsweise parallel zu einer Längsaxialrichtung A des Aufnahmekanals 14. Der Aufnahmekanal 14 ist im Querschnitt insbesondere kreisrund; gleiches gilt für den Anschlussstutzen 16.

Bevorzugt befindet sich die Kopplungsvorrichtung 6 an der Rückwand 38 des Behandlungsraums 36. Sie ist passend zu der Position des Verbindungselements 10 an dem Reinigungskorb 40 positioniert. Insbesondere sind also die Positionen des Verbindungselements 10 an dem Reinigungskorb 40 und die Position der Kopplungsvorrichtung 6 an der Rückwand 38 des Behandlungsraums 36 so durch die Führungen und Anschläge des Reinigungskorbs 40 aufeinander abgestimmt, dass beim Einschieben des Reinigungskorbs 40 die Anschlussstutzen 16 in den zugehörigen Aufnahmekanal 14 eingeschoben werden.

Vorteilhaft erfolgt diese Bewegung, ohne dass hierzu ein Widerstand einer Dichtsituation überwunden werden muss. Ein Durchmesser des Anschlussstutzens 16 und ein Durchmesser des Aufnahmekanals 14 sind unter Berücksichtigung ausreichender Toleranzen und Spaltmaße aufeinander abgestimmt.

Der Vorgang des Einschiebens des Anschlussstutzens 16 in den Aufnahmekanal 14 ist in den Fig. 1 bis 3 während verschiedener aufeinanderfolgender Phasen dargestellt.

Es ist insbesondere ein definierter Anschlag vorgesehen, um dem Anwender zu signalisieren, dass sich der Reinigungskorb 40 in der Endposition befindet. Die Endposition ist beispielsweise erreicht, wenn der Kragen des Verbindungselements 10 an der diesem zugewandten Flachseite der Aufnahme 6 anliegt.

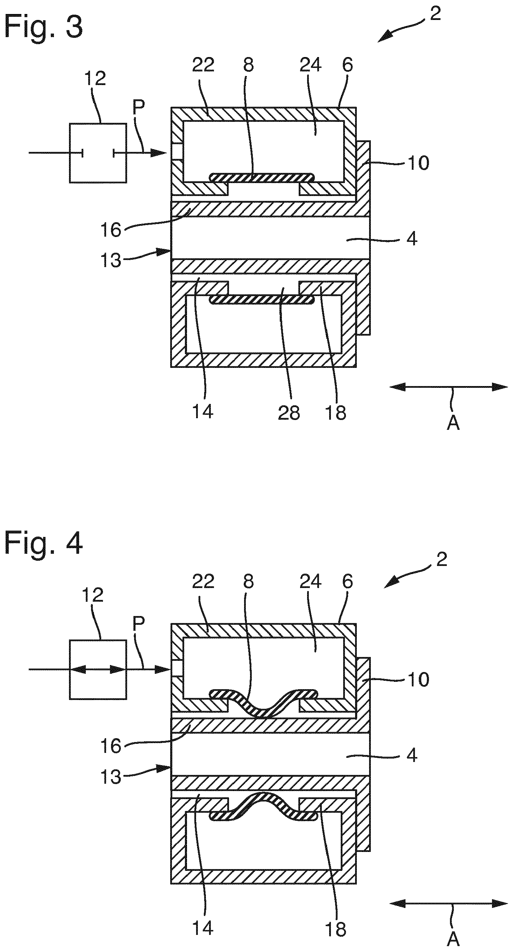

In der Endposition, wie sie Fig. 3 zeigt, ist noch kein flüssigkeitsdichter Kontakt zwischen der Kopplungsvorrichtung 6 und dem Verbindungselement 10 hergestellt. Insbesondere ist kein besonderer Kraftaufwand erforderlich gewesen, um die Endposition zu erreichen.

Eine flüssigkeitsdichte Verbindung zwischen dem Verbindungselement 10 und der Kopplungsvorrichtung 6 wird durch hydraulische und/oder pneumatische Betätigung des elastischen Dichtelements erreicht. Als Dichtelement ist gemäß dem dargestellten Ausführungsbeispiel eine Dichtmembran 8 vorgesehen. Zu deren Betätigung umfasst ein äußeres Bauteil 22 der Kopplungsvorrichtung 6 eine Überdruckkammer 24, die mit Hilfe der Druckluftversorgung 12 mit einem Überdruck beaufschlagbar ist. Zu diesem Zweck ist die Kopplungsvorrichtung 6 mit einer Druckluftleitung 46 mit der Druckluftversorgung 12 verbunden.

Die vorgesehene Dichtmembran 8 ist elastisch verformbar. Schließt nach Erreichen der Endposition der Anwender anschließend die Reinigungs- und/oder Desinfiziereinrichtung 30 und wählt ein entsprechendes Reinigungs- und/oder Desinfektionsprogramm, wird der Überdruckkammer 24 durch Aktivierung der Druckluftversorgung 12 Druckluft P zugeführt.

Die Membran 8 wird hydraulisch und/oder pneumatisch betätigt, wobei durch eine Expansion der Membran 8 in eine Richtung quer zu der Längsaxialrichtung A, ein Querschnitt des Aufnahmekanals 14 im Bereich der Membran 8 soweit verringert wird, dass eine flüssigkeitsdichte Verbindung des Verbindungselements 10 mit der Kopplungsvorrichtung 6 bereitstellbar ist oder bereitgestellt wird.

Das Dichtelement, d.h. die Membran 8, befindet sich an bzw. in einer Innenwand 18 der Überdruckkammer 24. Sie ist bei pneumatischer Betätigung, d.h. wenn in der Überdruckkammer 24 ein Überdruck hergestellt wird, expandierbar, so dass ein Dichtsitz zwischen der Membran 8 und einer Außenseite 20 des Anschlussstutzens 16 des Verbindungselements 10 herstellbar ist. Das äußere Bauteil 22 der Kopplungsvorrichtung 6 umfasst einen sich vollständig entlang des Umfangs des Aufnahmekanals 14 erstreckenden ringförmigen Spalt 28. Dieser entsteht durch eine ringförmige Unterbrechung der Innenwand 18, die sich zwischen dem Aufnahmekanal 14 und der Überdruckkammer 24 erstreckt. Der ringförmige Spalt 28 ist mit der elastischen Dichtmembran 8 als Dichtelement verschlossen. Wird die Überdruckkammer 24 mit einem Überdruck beaufschlagt, so wölbt sich die Dichtmembran 8 durch den ringförmigen Spalt 28 hindurch, so dass ein zuverlässiger Dichtsitz hergestellt wird. Diese Situation ist in Fig. 4 schematisch dargestellt.

Gemäß einem weiteren nicht dargestellten Ausführungsbeispiel ist eine ringförmige oder schlauchförmige Dichtung vorgesehen, die sich ebenfalls an der Innenwand 18 befindet und sich vollständig entlang dem Innenumfang des Aufnahmekanals 14 erstreckt. Auf eine Überdruckkammer 24 im Inneren des äußeren Bauteils 22 kann bei diesem Ausführungsbeispiel verzichtet werden. Der Innenraum der schlauchförmigen Dichtung übernimmt die technische Funktion der Überdruckkammer 24. Die Innenwand 18 ist gemäß diesem Ausführungsbeispiel insbesondere mit Mitteln zur Aufnahme und Halterung einer schlauchförmigen Dichtung versehen, beispielsweise weist sie umlaufende Nut auf. Im drucklosen Zustand legt sich eine solche schlauchförmige Dichtung flach an die Innenseite der Innenwand 18. Wird sie mit Druck beaufschlagt, so expandiert sie insbesondere unter Zunahme ihres Umfangs, so dass ein Dichtsitz zwischen der Dichtung und einer Außenseite 20 des Anschlussstutzens 16 des Verbindungselements 10 herstellbar ist.

Nach dem Zuführen von Druckluft P in die Überdruckkammer 24 des Anschlussstutzens 16 wird das Zuführdruckniveau der Druckluft P durch Zuschalten oder Abschalten der Druckluftversorgung 12 verändert bzw. für die Dauer des Reinigungs- und/oder Desinfiziervorgangs aufrechterhalten. Zu diesem Zweck wird der Druck in der Überdruckkammer 24 zeitabhängig gemessen, es ist ein entsprechender, nicht dargestellter, Sensor vorgesehen. Durch einen Vergleich mit einem vorgegebenen Sollwert wird der gewünschte Druck innerhalb der Überdruckkammer 24 aufrechterhalten.

So ist sichergestellt, dass für die Dauer des Reinigungs- und/oder Desinfiziervorgangs der Dichtsitz zwischen der Membran 8 und der Außenseite 20 des Verbindungselements 6 vorhanden ist. Nach Beendigung des Reinigungs- und/oder Desinfiziervorgangs wird die Druckluft P aus der Überdruckkammer 24 abgelassen, so dass die Kopplungsvorrichtung 6 das Verbindungselement 10 freigibt.

Um ein beschädigtes Dichtelement zu detektieren, ist die Reinigungs- und/oder Desinfiziereinrichtung 30 insbesondere dazu eingerichtet, Druckluft P der Überdruckkammer 24 mit einem Zuführdruckniveau zuzuführen, wobei das Zuführdruckniveau der Druckluft P durch Zuschalten oder Abschalten der Druckluftversorgung 12 verändert wird. Es wird der Druck an einem Eingang der Überdrucckammer 24 oder in einer Versorgungsleitung der Überdruckkammer 24 über eine erste vorgebbare Zeit gemessen. Das Zuführdruckniveau wird anschließend stufenförmig verändert. Eine Dichtigkeit der Überdruckkammer 24 wird anhand einer gemessenen Sprungantwort auf das Verändern des Zuführdruckniveaus detektiert, indem die Sprungantwort analysiert wird.

Diese Analyse umfasst insbesondere einen Vergleich eines statischen Drucks mit einem für die Überdruckkammer 24 vorgebbaren ersten Wert. Ferner umfasst diese Analyse insbesondere das Bilden eines Integrals des gemessenen Drucks über eine zweite vorgebbare Zeit und einen Vergleich mit einem für die Überdruckkammer 24 vorgebbaren zweiten Wert. Außerdem umfasst die Analyse insbesondere das Bestimmen einer Steigung und/oder das Bilden einer zweiten Ableitung des gemessenen Drucks nach der Zeit. Schließlich umfasst die Analyse insbesondere die Analyse eine Dauer, Amplitude und/oder Wellenlänge einer Schwingung des als Sprungantwort gemessenen Drucks.

Übersteigt der Druckabfall einen gewissen Grenzwert, so wird dies als Hinweis auf ein defektes Dichtelement, insbesondere eine defekte Dichtmembran 8, gewertet. Die Reinigungs- und/oder Desinfiziereinrichtung 30 sind insbesondere dazu eingerichtet, ein entsprechendes Fehler- oder Hinweissignal auszugeben, welches zur Wartung oder zum Austausch des Dichtelements auffordert.

Ferner ist es möglich, das Vorhandensein oder Nicht-Vorhandensein des Anschlussstutzens 16 des Verbindungselements 10 in dem Aufnahmekanal 14 anhand der elastischen Reaktion der Dichtmembran 8 festzustellen. Wird der Innendruck in der Überdruckkammer 24 verändert, so zeigt die Dichtmembran 8 ein unterschiedliches elastisches Verhalten, abhängig davon, ob der Anschlussstutzen 16 in dem Aufnahmekanal 14 vorhanden ist oder nicht.

So übt beispielsweise der Anschlussstutzen 16 eine Gegenkraft auf die Dichtmembran 8 aus und begrenzt außerdem das zur elastischen Verformung bzw. Ausdehnung zur Verfügung stehende Volumen. Insbesondere durch eine Analyse einer Steigung und/oder das Bilden einer zweiten Ableitung des zeitabhängig gemessenen Drucks, kann festgestellt werden, ob sich der Anschlussstutzen 16 in dem Aufnahmekanal 14 befindet oder nicht. Ferner bietet sich zu diesem Zweck eine Analyse einer Dauer, einer Amplitude und/oder einer Wellenlänge einer Schwingung des als Sprungantwort auf eine Veränderung des Innendrucks gemessenen Drucks an.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezuqszeichenliste

- 2: Verbindungseinrichtung
- 4: Verbindungskanal
- 5: Spülflüssigkeit
- 6: Kopplungsvorrichtung
- 7: Spülflüssigkeitsvorrat
- 8: Dichtmembran
- 10: Verbindungselement
- 12: Druckluftversorgung
- 13: Versorgungsöffnung
- 14: Aufnahmekanal
- 16: Anschlussstutzen
- 18: Innenwand
- 20: Außenseite
- 22: äußeres Bauteil
- 24: Überdruckkammer
- 26: Trennwand
- 28: ringförmiger Spalt
- 30: Reinigungs- und/oder Desinfiziereinrichtung
- 32: Endoskop
- 34: Endoskopkanal
- 36: Reinigungsraum
- 38: Wand
- 40: Reinigungskorb
- 42: Versorgungsleitung
- 44: Verbindungseinrichtung
- 46: Druckluftleitungen
- 50: Spülsystem
- P: Druckluft
- A: Längsaxialrichtung

## Patentansprüche

1. Verbindungseinrichtung (2) zum Ankoppeln eines Spülsystems (50) an einen zu reinigenden Kanal eines chirurgischen Instruments, insbesondere einen Endoskopkanal (34) eines Endoskops (32), wobei die Verbindungseinrichtung (2) eine versorgungsseitige Kopplungsvorrichtung (6) mit einem Aufnahmekanal (14) umfasst, der dazu eingerichtet ist, ein Verbindungselement (10) aufzunehmen, welches sich im verbundenen Zustand in einer Längsaxialrichtung (A) des Aufnahmekanals (14) innerhalb des Aufnahmekanals (14) erstreckt und welches endseitig eines Verbindungskanals (4) vorgesehen ist, der mit dem zu reinigenden Kanal des chirurgischen Instruments kommuniziert, wobei der Aufnahmekanal (14) zur Zuführung von Spülflüssigkeit (5) zu dem zu reinigenden Kanal mit dem Spülsystem (50) verbunden ist, wobei die Kopplungsvorrichtung (6) ein Dichtelement umfasst, wobei das Dichtelement elastisch ist und Mittel zur hydraulischen und/oder pneumatischen Betätigung des Dichtelements vorgesehen sind, wobei das hydraulisch und/oder pneumatisch betätigte Dichtelement durch eine Expansion quer zu der Längsaxialrichtung (A) einen Querschnitt des Aufnahmekanals (14) im Bereich des Dichtelements verringert, so dass eine flüssigkeitsdichte Verbindung des Verbindungselements (10) mit der Kopplungsvorrichtung (6) bereitstellbar ist oder bereitgestellt wird, **dadurch gekennzeichnet, dass**
eine Überdruckkammer (24) und eine Druckluftversorgung (12) vorgesehen sind, wobei die Druckluftversorgung (12) mit der Überdruckkammer (24) verbunden ist und das Dichtelement zumindest einen Wandabschnitt der Überdruckkammer (24) bildet,
die Kopplungsvorrichtung (6) die Überdruckkammer (24) umfasst, wobei sich die Überdruckkammer (24) entlang einem Au-βenumfang des Aufnahmekanals (14) erstreckt und wobei das Dichtelement eine Dichtmembran (8) ist, die die Überdrucckammer (24) an einer dem Aufnahmekanal (14) zugewandten Innenwand (18) der Überdruckkammer (24) begrenzt und
ein äußeres Bauteil (22) der Kopplungsvorrichtung (6) eine mit einer Druckluftversorgung (12) verbundene Überdruckkammer (24) umfasst, wobei die Innenwand (18) der Überdruckkammer (24) entlang dem Umfang des Aufnahmekanals (14) ringförmig unterbrochen ist, so dass die Innenwand (18) einen ringförmigen Spalt (28) aufweist, wobei der ringförmige Spalt (28) mit der elastischen Dichtmembran (8) als Dichtelement verschlossen ist.

2. Verbindungseinrichtung (2) nach Anspruch 1, **dadurch gekennzeichnet, dass** die Überdruckkammer (24) vollständig entlang dem Umfang des Aufnahmekanals (14) verläuft, wobei durch Beaufschlagen der Überdruckkammer (24) mit einem Überdruck eine flüssigkeitsdichte Ankopplung des Verbindungselements (10) an die Kopplungsvorrichtung (6) bereitstellbar ist oder bereitgestellt wird, indem ein Dichtsitz zwischen dem expandierten, die Innenwand (18) der Überdruckkammer (24) überragenden und so den Querschnitt des Aufnahmekanals (14) lokal reduzierenden Dichtelements und einer Außenseite (20) eines sich innerhalb des Aufnahmekanals (14) erstreckenden Anschlussstutzens (16) des Verbindungselements (10) herstellbar ist oder hergestellt wird.

3. Reinigungs- und/oder Desinfiziereinrichtung (30) zur Reinigung und/oder Desinfektion von chirurgischen Instrumenten, insbesondere Endoskopen (32), mit einem zu reinigenden Kanal, insbesondere zur Reinigung und/oder Desinfektion von Endoskopen (32) mit einem zu reinigenden Endoskopkanal (34), umfassend einen Reinigungsraum (36), in dem die zu reinigenden und/oder zu desinfizierenden chirurgischen Instrumente, insbesondere Endoskope (32), während eines Reinigungs- und/oder Desinfektionsvorgangs aufnehmbar sind oder aufgenommen werden, **dadurch gekennzeichnet, dass** eine Verbindungseinrichtung (2) nach Anspruch 1 oder 2 vorgesehen ist, die insbesondere an einer Wand (38) des Reinigungsraums (36) angeordnet oder in eine Wand (36) des Reinigungsraums (36) eingelassen ist.

4. Verfahren zum Betreiben einer Reinigungs- und/oder Desinfiziereinrichtung (30) nach Anspruch 3, mit den folgenden Verfahrensschritten :
- Einbringen eines Anschlussstutzens (16) des Verbindungselements (10) entlang der Längsaxialrichtung (A) des Aufnahmekanals (14) in den Aufnahmekanal (14) der versorgungsseitigen Kopplungsvorrichtung (6),
- Anlegen eines Überdrucks an das Dichtelement durch Beaufschlagen des Dichtelements mit einem unter Überdruck stehenden hydraulisch und/oder pneumatisch wirksamen Fluid zur hydraulischen und/oder pneumatischen Betätigung des Dichtelements, so dass das betätigte Dichtelement durch eine Expansion quer zu der Längsaxialrichtung (A) einen Querschnitt des Aufnahmekanals (14) im Bereich des Dichtelements verringert,
- Beaufschlagen des Aufnahmekanals (14) mit einer Spülflüssigkeit (5), so dass die Spülflüssigkeit (5) ausgehend von dem Aufnahmekanal (14), über den Verbindungskanal (4) zum Reinigen und/oder zum Desinfizieren des zu reinigenden Kanals des chirurgischen Instruments, insbesondere zum Reinigen und/oder zum Desinfizieren eines Endoskopkanals (34) eines Endoskops (32), zur Verfügung steht,
- Entlasten des hydraulisch und/oder pneumatisch wirksamen Fluids, um den durch das Fluid auf das Dichtelement wirkenden Überdruck abzubauen, so dass das Verbindungselement (10) von der Kopplungsvorrichtung (6) freigegeben wird.

5. Verfahren nach Anspruch 4, **dadurch gekennzeichnet, dass** die folgenden weiteren Schritte vorgesehen sind:
- Zuführen von Druckluft (P) zu einer zumindest teilweise von dem Dichtelement umschlossenen Überdruckkammer (24), wobei ein Zuführdruckniveau der Druckluft (P) durch Zuschalten oder Abschalten einer Druckluftversorgung (12) verändert wird,
- Messen eines zeitabhängigen Drucks in der Überdruckkammer (24) und Vergleichen des gemessenen Drucks mit einem vorgegebenen Solldruck, wobei durch Zuschalten oder Abschalten der Druckluftversorgung (12) der Solldruck für eine Dauer des Reinigungs- und/oder Desinfiziervorgangs aufrechterhalten wird.

6. Verfahren nach Anspruch 4 oder 5, **dadurch gekennzeichnet, dass** der Druck in der Überdruckkammer (24) an einem mit einer Druckluftversorgung(12) verbundenen Zugang der Überdruckkammer (24) über einen ersten Zeitraum gemessen wird, wobei anschließend das Zuführdruckniveau stufenförmig verändert wird und eine Sprungantwort des in der Überdruckkammer (24) herrschenden Drucks auf das Verändern des Zuführdruckniveaus gemessen und analysiert wird, wobei die Analyse Auskunft über eine Dichtigkeit der Überdruckkammer (24), insbesondere eine Dichtigkeit des Dichtelements, und/oder Auskunft über ein Vorhandensein oder Nicht-Vorhandensein des Anschlussstutzens (16) des Verbindungselements (10) in dem Aufnahmekanal (14) gibt.

7. Verfahren nach Anspruch 6, **dadurch gekennzeichnet, dass** die Analyse das Bilden eines Integrals des gemessenen Drucks über eine vorgebbare Zeit, das Bestimmen einer Steigung und/oder das Bilden einer zweiten Ableitung des als Sprungantwort gemessenen Drucks nach der Zeit, beinhaltet, wobei ein Vergleich mit einem vorgegebenen Sollwert durchgeführt wird und bei Über- oder Unterschreiten des vorgegebenen Sollwerts auf eine nicht ausreichende Dichtigkeit der Überdruckkammer (24), insbesondere des Dichtelements, und/oder auf ein Vorhandensein oder Nicht-Vorhandensein des Anschlussstutzens (16) des Verbindungselements (10) in dem Aufnahmekanal (14) geschlossen wird.

## Claims

1. A connecting device (2) for coupling a rinsing system (50) to a channel of a surgical instrument, which channel is to be cleaned, in particular an endoscope channel (34) of an endoscope (32), wherein the connecting device (2) comprises a supply-side coupling device (6) having an accommodating channel (14), which accommodating channel is designed to accommodate a connecting element (10) which extends within the accommodating channel (14) in a longitudinal axial direction (A) of the accommodating channel (14) in the connected state and which is provided at the end of a connecting channel (4) which communicates with the channel of the surgical instrument, which channel is to be cleaned, wherein the accommodating channel (14) is connected to the rinsing system (50) in order to feed rinsing liquid (5) to the channel to be cleaned, wherein the coupling device (6) comprises a sealing element, wherein the sealing element is elastic and means for hydraulically and/or pneumatically actuating the sealing element are provided, wherein the hydraulically and/or pneumatically actuated sealing element reduces a cross-section of the accommodating channel (14) in the region of the sealing element by means of an expansion transverse to the longitudinal axial direction (A) such that a liquid-tight connection of the connecting element (10) to the coupling device (6) can be or is provided, **characterized in that**
an overpressure chamber (24) and a compressed air supply (12) are provided, wherein the compressed air supply (12) is connected to the overpressure chamber (24), and the sealing element forms at least one section of a wall of the overpressure chamber (24), the coupling device (6) comprises the overpressure chamber (24), wherein the overpressure chamber (24) extends along an outer perimeter of the accommodating channel (14), and wherein the sealing element is a sealing membrane (8) which delimits the overpressure chamber (24) at an inner wall (18) of the overpressure chamber (24), the inner wall facing the accommodating channel (14), and
an outer component (22) of the coupling device (6) comprises an overpressure chamber (24) connected to a compressed air supply (12), wherein the inner wall (18) of the overpressure chamber (24) is interrupted in a ring-shaped manner along the perimeter of the accommodating channel (14) such that the inner wall (18) has an annular gap (28), wherein the annular gap (28) is sealed with the elastic sealing membrane (8) as the sealing element.

2. The connecting device (2) according to Claim 1, **characterized in that** the overpressure chamber (24) runs completely along the perimeter of the accommodating channel (14), wherein by applying an overpressure to the overpressure chamber (24), a liquid-tight coupling of the connecting element (10) to the coupling device (6) can be or is provided in that a sealing seat can be or is established between the expanded sealing element that extends beyond the inner wall (18) of the overpressure chamber (24) and thus locally reduces the cross-section of the accommodating channel (14), and an outside (20) of a coupling (16) of the connecting element (10) extending within the accommodating channel (14).

3. A cleaning and/or disinfecting device (30) for cleaning and/or disinfecting surgical instruments, in particular endoscopes (32), having a channel to be cleaned, in particular for cleaning and/or disinfecting endoscopes (32) having an endoscope channel (34) to be cleaned, comprising a cleaning chamber (36), in which the surgical instruments to be cleaned and/or to be disinfected, in particular endoscopes (32), can be or are accommodated during a cleaning and/or disinfection process, **characterized in that** a connecting device (2) according to Claim 1 or 2 is provided, which is arranged in particular on a wall (38) of the cleaning chamber (36) or is inserted into a wall (36) of the cleaning chamber (36).

4. A method for operating a cleaning and/or disinfecting device (30) according to Claim 3, having the following method steps:
- introducing a coupling (16) of the connecting element (10) along the longitudinal axial direction (A) of the accommodating channel (14) into the accommodating channel (14) of the supply-side coupling device (6),
- applying an overpressure to the sealing element by supplying hydraulic and/or pneumatic fluid subject to an overpressure to the sealing element to hydraulically and/or pneumatically actuate the sealing element such that the actuated sealing element reduces a cross-section of the accommodating channel (14) in the region of the sealing element by means of an expansion transverse to the longitudinal axial direction (A),
- supplying rinsing liquid (5) to the accommodating channel (14) such that the rinsing liquid (5), starting from the accommodating channel (14), is available via the connecting channel (4) for cleaning and/or for disinfecting the channel of the surgical instrument, which channel is to be cleaned, in particular for cleaning and/or for disinfecting an endoscope channel (34) of an endoscope (32),
- releasing the pressure from the hydraulic and/or pneumatic fluid in order to decrease the overpressure acting on the sealing element by the fluid such that the connecting element (10) is released from the coupling device (6).

5. The method according to Claim 4, **characterized in that** the following further steps are provided:
- supplying compressed air (P) to an overpressure chamber (24) at least partially enclosed by the sealing element, wherein a supply pressure level of the compressed air (P) is changed by turning on or turning off a compressed air supply (12),
- measuring a time-dependent pressure in the overpressure chamber (24) and comparing the measured pressure with a predefined target pressure, wherein by turning on or turning off the compressed air supply (12), the target pressure is maintained for a duration of the cleaning and/or disinfecting process.

6. The method according to Claim 4 or 5, **characterized in that** the pressure in the overpressure chamber (24) is measured at an access to the overpressure chamber (24) connected to a compressed air supply (12) over a first period of time, wherein the supply pressure level is then changed in steps and a step response of the prevailing pressure in the overpressure chamber (24) to the change in the supply pressure level is measured and analyzed, wherein the analysis provides information on a sealing of the overpressure chamber (24), in particular a sealing of the sealing element, and/or information on a presence or absence of the coupling (16) of the connecting element (10) in the accommodating channel (14).

7. The method according to Claim 6, **characterized in that** the analysis contains the formation of an integral of the measured pressure over a predefinable time, the determination of a rise and/or the formation of a second derivative of the pressure measured as a step response over time, wherein a comparison with a predefined target value is performed, and if the predefined target value is exceeded or fallen short of, an insufficient sealing of the overpressure chamber (24), in particular of the sealing element, and/or a presence or absence of the coupling (16) of the connecting element (10) in the accommodating channel (14) is assumed.

## Revendications

1. Dispositif de raccordement (2) destiné à l'accouplement d'un système de rinçage (50) à un canal à nettoyer d'un instrument chirurgical, en particulier à un canal d'endoscope (34) d'un endoscope (32), le dispositif de raccordement (2) comportant un système d'accouplement (6) côté alimentation avec un canal de réception (14) conçu pour recevoir un élément de raccordement (10) lequel s'étend, à l'état raccordé, à l'intérieur du canal de réception (14) dans une direction axiale longitudinale (A) du canal de réception (14) et lequel est prévu du côté de l'extrémité d'un canal de raccordement (4) communiquant avec le canal à nettoyer de l'instrument chirurgical, dans lequel le canal de réception (14) est relié au système de rinçage (50) pour l'alimentation de liquide de rinçage (5) vers le canal à nettoyer, dans lequel le système d'accouplement (6) comporte un élément d'étanchéité, dans lequel l'élément d'étanchéité est élastique et dans lequel il est prévu des moyens d'actionnement hydraulique et/ou pneumatique de l'élément d'étanchéité, dans lequel l'élément d'étanchéité actionné de façon hydraulique et/ou pneumatique réduit une section transversale du canal de réception (14) dans la région de l'élément d'étanchéité du fait d'une dilatation transversalement à la direction axiale longitudinale (A), de manière à pouvoir fournir ou à fournir un raccordement étanche aux liquides de l'élément de raccordement (10) au système d'accouplement (6), **caractérisé en ce que**
- il est prévu une chambre de surpression (24) et une alimentation en air comprimé (12), l'alimentation en air comprimé (12) étant reliée à la chambre de surpression (24) et l'élément d'étanchéité formant au moins une section de paroi de la chambre de surpression (24),
- le système d'accouplement (6) comporte la chambre de surpression (24), la chambre de surpression (24) s'étendant le long d'un pourtour extérieur du canal de réception (14) et l'élément d'étanchéité étant une membrane d'étanchéité (8) délimitant la chambre de surpression (24) au niveau d'une paroi intérieure (18) de la chambre de surpression (24) tournée vers le canal de réception (14), et
- une pièce de construction extérieure (22) du système d'accouplement (6) comporte une chambre de surpression (24) reliée à l'alimentation en air comprimé (12), la paroi intérieure (18) de la chambre de surpression (24) étant interrompue de façon annulaire le long du pourtour du canal de réception (14), de telle façon que la paroi intérieure (18) présente une fente annulaire (28), la fente annulaire (28) étant fermée par la membrane d'étanchéité (8) élastique en tant qu'élément d'étanchéité.

2. Dispositif de raccordement (2) selon la revendication 1, **caractérisé en ce que** la chambre de surpression (24) se prolonge entièrement le long du pourtour du canal de réception (14), dans lequel le fait de soumettre la chambre de surpression (24) à une surpression permet de fournir ou fournit un accouplement étanche au liquide de l'élément de raccordement (10) au système d'accouplement (6), en pouvant réaliser ou réalisant un siège étanche entre l'élément d'étanchéité dilaté faisant saillie au-delà de la paroi intérieure (18) de la chambre de surpression (24) et réduisant ainsi localement la section transversale du canal de réception (14) et un côté extérieur (20) d'un embout de connexion (16) de l'élément de raccordement (10) s'étendant à l'intérieur du canal de réception (14).

3. Dispositif de nettoyage et/ou de désinfection (30) destiné à nettoyer et/ou à désinfecter des instruments chirurgicaux, en particulier des endoscopes (32), avec un canal à nettoyer, en particulier pour le nettoyage et/ou la désinfection d'endoscopes (32) avec un canal d'endoscope (34) à nettoyer, comportant une chambre de nettoyage (36) dans laquelle peuvent être reçus ou sont reçus les instruments chirurgicaux à nettoyer et/ou à désinfecter, en particulier des endoscopes (32), au cours d'une opération de nettoyage et/ou de désinfection, **caractérisé en ce qu'**il est prévu un dispositif de raccordement (2) selon la revendication 1 ou 2, lequel est en particulier disposé sur une paroi (38) de la chambre de nettoyage (36) ou encastré dans une paroi (36) de la chambre de nettoyage (36).

4. Procédé de fonctionnement d'un dispositif de nettoyage et/ou de désinfection (30) selon la revendication 3, comprenant les étapes suivantes :
- introduction d'un embout de connexion (16) de l'élément de raccordement (10) le long de la direction axiale longitudinale (A) du canal de réception (14) dans le canal de réception (14) du système d'accouplement (6) côté alimentation,
- application d'une surpression à l'élément d'étanchéité par sollicitation de l'élément d'étanchéité avec un fluide soumis à une surpression et agissant de façon hydraulique et/ou pneumatique, pour l'actionnement hydraulique et/ou pneumatique de l'élément d'étanchéité, de manière à ce que l'élément d'étanchéité actionné réduise une section transversale du canal de réception (14) dans la région de l'élément d'étanchéité par une dilatation transversalement à la direction axiale longitudinale (A),
- sollicitation du canal de réception (14) avec un liquide de rinçage (5), de manière à ce que le liquide de rinçage (5) soit rendu disponible à partir du canal de réception (14), en passant par le canal de raccordement (4), pour le nettoyage et/ou la désinfection du canal à nettoyer de l'instrument chirurgical, en particulier pour le nettoyage et/ou la désinfection d'un canal d'endoscope (34) d'un endoscope (32),
- déchargement du fluide agissant de façon hydraulique et/ou pneumatique, afin de diminuer la surpression agissant sur l'élément d'étanchéité par le biais du fluide, de manière à libérer l'élément de raccordement (10) par rapport au système d'accouplement (6).

5. Procédé selon la revendication 4, **caractérisé en ce que** les étapes supplémentaires suivantes sont prévues :
- approvisionnement d'air comprimé (P) vers une chambre de surpression (24) au moins partiellement entourée par l'élément d'étanchéité, un niveau de pression d'approvisionnement de l'air comprimé (P) étant modifié par ajout ou arrêt d'une alimentation en air comprimé (12),
- mesure d'une pression dépendante du temps dans la chambre de surpression (24) et comparaison de la pression mesurée avec une pression de consigne prédéfinie, la pression de consigne étant maintenue pour une durée de l'opération de nettoyage et/ou de désinfection par l'ajout ou l'arrêt d'une alimentation en air comprimé (12).

6. Procédé selon la revendication 4 ou 5, **caractérisé en ce que** la pression dans la chambre de surpression (24) est mesurée sur une première durée au niveau d'un accès de la chambre de surpression (24) relié à l'alimentation en air comprimé (12), dans lequel le niveau de pression d'approvisionnement est ensuite modifié par paliers et une réponse de saut de la pression régnant dans la chambre de surpression (24) à la modification du niveau de pression d'approvisionnement étant mesurée et analysée, dans lequel l'analyse donne des informations sur une étanchéité de la chambre de surpression (24), en particulier une étanchéité de l'élément d'étanchéité, et/ou des informations sur une présence ou une absence de l'embout de connexion (16) de l'élément de raccordement (10) dans le canal de réception (14).

7. Procédé selon la revendication 6, **caractérisé en ce que** l'analyse contient la formation d'une intégrale de la pression mesurée sur une durée prédéfinissable, la détermination d'une augmentation et/ou la formation d'une deuxième déviation de la pression mesurée en tant que réponse de saut après ladite durée, dans lequel une comparaison avec une valeur de consigne prédéfinie est effectuée, et une étanchéité insuffisante de la chambre de surpression (24), en particulier de l'élément d'étanchéité, et/ou une présence ou une absence de l'embout de connexion (16) de l'élément de raccordement (10) dans le canal de réception (14) est déduite lorsque la valeur de consigne prédéfinie est dépassée ou n'est pas atteinte.
